# EUROPEAN PATENT SPECIFICATION

(11) **EP 2 574 271 B1**
(45) Date of publication and mention of the grant of the patent: **05.11.2014**
(21) Application number: 12181878.5
(22) Date of filing: 27.08.2012
(51) Int. Cl.: A61B 1/00

(54) **Closure device for end opening**
Verschlussvorrichtung für eine Endöffnung
Dispositif de fermeture pour ouverture d'extrémité

(30) Priority: 30.09.2011 JP 2011216681
(43) Date of publication of application: 03.04.2013
(73) Proprietor: Fujifilm Corporation, Minato-ku Tokyo (JP)
(72) Inventor: Yamane, Kenji, Kanagawa (JP)
(74) Representative: Klunker . Schmitt-Nilson . Hirsch

(56) References cited:
- US-A1- 2010 087 710
- US-A1- 2010 198 007

## Description

### BACKGROUND OF THE INVENTION

### 1. Field of the Invention

The present invention relates to a closure device for an end opening. More particularly, the present invention relates to a closure device for an end opening of an endoscope, in which a medical instrument is guided through its guide lumen, and leakage of fluid can be prevented reliably even upon removal of the medical instrument.

### 2. Description Related to the Prior Art

An endoscope includes an elongated tube, a tip device and an imaging window. The elongated tube is entered in a body cavity of a patient. The tip device is disposed at a distal end of the elongated tube. The imaging window is disposed on a distal surface of the tip device, and receive obj ect light from an obj ect of interest for imaging. Also, an instrument channel is formed through the elongated tube. A medical instrument, such as a forceps, is entered through the instrument channel for a doctor medically to treat a lesion at the object of interest in the body.

An end opening of the instrument channel is formed in a handle at a proximal end of the elongated tube. A closure device closes the end opening. The closure device includes a guide lumen and sealing walls. The guide lumen receives entry of a medical instrument. The sealing walls tightly close the guide lumen so as to prevent leakage of body fluid and the like when the medical instrument is not present.

JP-A 2006-288780 discloses the sealing walls, which normally close the guide lumen, and are pushed open by the medical instrument for use in the treatment.

However, there is a problem in JP-A 2006-288780 in that the sealing walls are bent back upon removal of the medical instrument to offset its edge, so that fluid is very likely to leak.

An alternative approach is described in US 2010/087710, where a seal member having an aperture for insertion of a medical instrument in a sealed manner is described.

### SUMMARY OF THE INVENTION

In view of the foregoing problems, an object of the present invention is to provide a closure device for an end opening of an endoscope, in which a medical instrument is guided through its guide lumen, and leakage of fluid can be prevented reliably even upon removal of the medical instrument.

In order to achieve the above and other objects and advantages of this invention, a closure device for an end opening of an instrument channel for entry of an instrument is provided. There is a coupling sleeve for connection with the end opening, the coupling sleeve having a guide lumen for passage of the instrument. At least two sealing walls are disposed to project from the coupling sleeve in a proximal direction in relation to the entry of the instrument, shiftable between open and closed positions, for tightly contacting one another to seal the guide lumen when in the closed position, the sealing walls being shifted in the open position by the instrument being entered, for preventing fluid from passing around the instrument. A housing sleeve contains at least the sealing walls. A coupling flange is disposed at an end of the sealing walls in the proximal direction, for mounting the sealing walls in the housing sleeve. A first regulating projection is disposed to project from an inner surface of the housing sleeve, for regulating movement of the coupling sleeve in the proximal direction. A second regulating projection is disposed to project from the inner surface of the housing sleeve, for regulating movement of the coupling flange in a distal direction.

Each of the sealing walls includes a sealing surface for contact in relation to the closed position. A first inclined surface is disposed to extend from the sealing surface to the coupling sleeve. A second inclined surface is disposed to extend from the sealing surface to the coupling flange, for guiding the instrument.

The second inclined surface might be substantially flat, and an inner width thereof between the sealing walls might increase toward the coupling flange.

In a preferred embodiment, the second inclined surface is curved, and an inner diameter thereof increases toward the coupling flange.

The sealing walls and the coupling sleeve constitute a sealing unit.

Furthermore, an internal space is disposed between the housing sleeve and the sealing walls, for allowing the sealing walls to shift to the open position.

The first regulating projection is disposed on a distal side from the sealing surface, the second regulating projection is disposed on a proximal side from the sealing surface, and the internal space is defined between the first and second regulating projections.

Furthermore, a recess is formed in the sealing walls with an outer surface back to the sealing surface, for constituting the internal space.

Furthermore, a pressing unit presses the sealing walls toward the closed position.

The pressing unit is the first regulating projection.

In a preferred embodiment, the pressing unit is the second regulating projection.

Furthermore, a pair of reinforcing walls are disposed on lateral sides of the plural sealing walls, for preventing occurrence of breakage by spacing between the sealing walls according to entry of the instrument.

Furthermore, a port plate is disposed in the housing sleeve on the proximal side of the sealing walls. An access hole is formed in the port plate, for receiving passage of the instrument. An inclined surface is formed with the access hole to decrease an inner width toward the sealing walls, for guiding the instrument.

The end opening has an end sleeve. Furthermore, a receiving groove is disposed between the housing sleeve and the coupling sleeve, formed at a distal end thereof, for receiving a sleeve edge of the end sleeve.

The end opening further includes a peripheral groove formed around the end sleeve and near to a proximal end thereof. Furthermore, an internal projection is formed on the housing sleeve to project from an inner surface of the receiving groove, engaged with the peripheral groove, for coupling to the end opening.

Also, an endoscope is provided, and includes an elongated tube for imaging in a body cavity. An instrument channel is formed through the elongated tube, for entry of an instrument. There is a closure device for an end opening of the instrument channel. The closure device includes a coupling sleeve for connection with the end opening, the coupling sleeve having a guide lumen for passage of the instrument. At least two sealing walls, disposed to project from the coupling sleeve in a proximal direction in relation to the entry of the instrument, shiftable between open and closed positions, for tightly contacting one another to seal the guide lumen when in the closed position, the sealing walls being shifted in the open position by the instrument being entered, for preventing fluid from passing around the instrument. A housing sleeve contains at least the sealing walls. A coupling flange is disposed to project from the sealing walls in the proximal direction, for coupling the sealing walls to the housing sleeve. A first regulating projection is disposed to project from an inner surface of the housing sleeve, for regulating the coupling sleeve in the proximal direction. A second regulating projection is disposed to project from the inner surface of the housing sleeve, for regulating the coupling flange in a distal direction.

Consequently, leakage of fluid can be prevented reliably even upon removal of the medical instrument, because the regulating projections is used for regulation and positioning the sealing walls in tight contact.

### BRIEF DESCRIPTION OF THE DRAWINGS

The above objects and advantages of the present invention will become more apparent from the following detailed description when read in connection with the accompanying drawings, in which:
Fig. 1 is a plan illustrating an endoscope system;
Fig. 2 is a vertical section illustrating a closure device;
Fig. 3A is a front perspective illustrating the port plate for entry;
Fig. 3B is a rear perspective illustrating the port plate;
Figs. 4A and 4B are perspectives illustrating a sealing unit in the closure device;
Figs. 5A and 5B are side elevations illustrating the sealing unit;
Figs. 6A and 6B are vertical sections illustrating the sealing unit;
Fig. 6C is a cross section illustrating the sealing unit;
Figs. 7A-7C are perspectives illustrating another preferred sealing unit;
Figs. 8A and 8B are side elevations illustrating the sealing unit;
Figs. 9A and 9B are vertical sections illustrating the sealing unit;
Fig. 9C is a cross section illustrating the sealing unit.

### DETAILED DESCRIPTION OF THE PREFERRED EMBODIMENT(S) OF THE PRESENT INVENTION

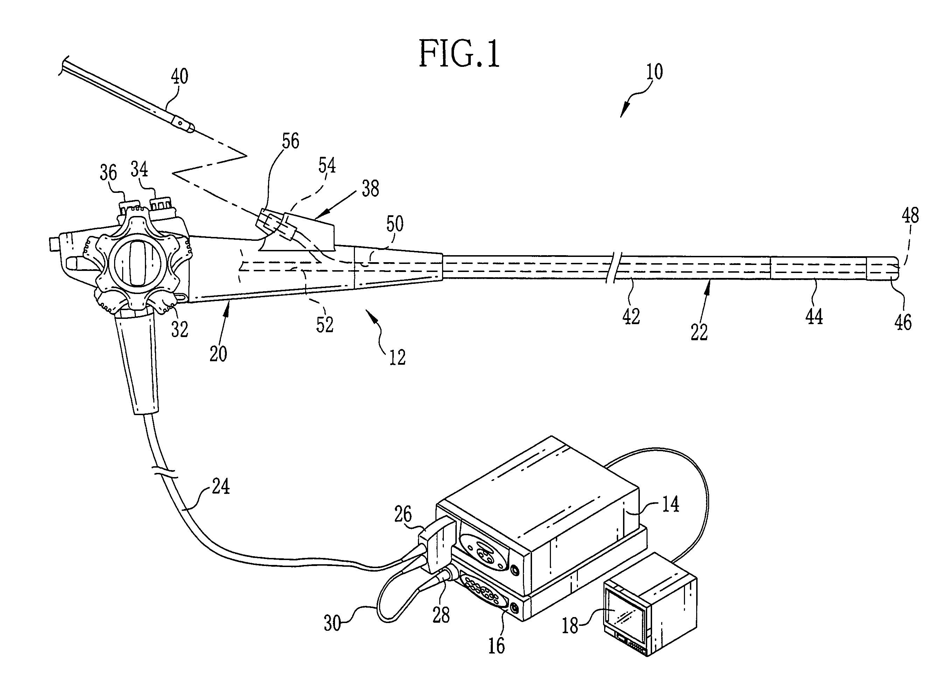

In Fig. 1, an endoscope system 10 includes an endoscope 12, a light source apparatus 14, a processing apparatus 16, and a monitor display panel 18. The endoscope 12 includes a handle device 20, an elongated tube 22 or guide tube, and a universal cable 24. Connection devices 26 and 28 and a signal cable 30 are used to connect the universal cable 24 to the light source apparatus 14 and to the processing apparatus 16.

The handle device 20 includes steering wheels 32, a fluid supply button 34 and a suction button 36. An end opening 38 or proximal opening is formed in the handle device 20 and near to the elongated tube 22. A medical instrument 40 is entered in the end opening 38, such as a forceps, electrocautery device, injection needle, and the like.

The elongated tube 22 extends from the handle device 20 and is entered in a body cavity of a patient. The elongated tube 22 includes a flexible device 42, a steering device 44 and a tip device 46 or head assembly. The flexible device 42 has such a sufficient length as to direct the tip device 46 to an object of interest in the body cavity. The steering device 44 is bendable to the right and left and up and down in response to operation of the steering wheels 32 of the handle device 20, to orient the tip device 46 in a direction desired by a doctor or operator.

Various elements are disposed on a distal surface of the tip device 46, including an imaging window, lighting windows, a nozzle spout of a fluid nozzle (all not shown). A distal opening 48 is formed in the distal surface of the tip device 46. A lens system and an imaging unit with a CCD or CMOS imaging sensor are disposed behind the imaging window. An image signal from the imaging unit is sent by the universal cable 24 to the processing apparatus 16. The processing apparatus 16 processes the image signal in image processing, converts the image signal into a video signal, and drives the display panel 18 to display an image.

A light guide device has a distal tip disposed at each one of the lighting windows. The light guide device guides light from the light source apparatus 14 to the lighting windows. The light is applied to an object of interest in a body cavity. The fluid nozzle supplies fluid toward the imaging window from a fluid supply source (not shown) in response to depression of the fluid supply button 34, the fluid being air and washing liquid.

An instrument channel 50 or working channel is disposed to extend through the elongated tube 22. The distal opening 48 is an end of the instrument channel 50, which communicates to the end opening 38 on a proximal side. A suction channel 52 is a branch of the instrument channel 50 near to the end opening 38. The suction channel 52 is connected with the suction button 36. The universal cable 24 connects the suction button 36 to a suction pump (not shown). The suction pump is always driven for operating the endoscope 12. When the suction button 36 is depressed, fluid is sucked through the suction channel 52 and the instrument channel 50, such as blood, body fluid, waste fluid, washing water and the like.

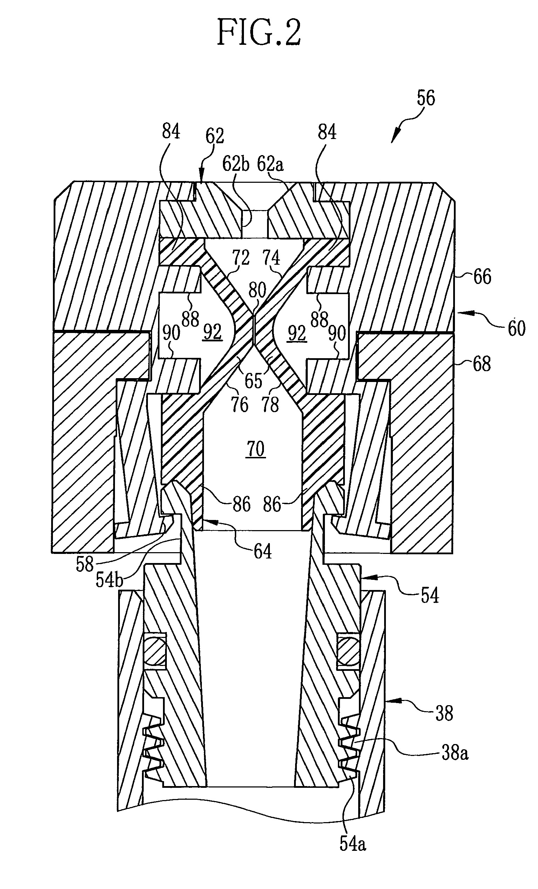

In Fig. 2, an end sleeve 54 is fitted in the end opening 38 to protrude in a sleeve form. A closure device 56 is mounted on the elongated tube 22 and closes the end sleeve 54. A male thread 54a is disposed around a tip of the end sleeve 54. A female thread 38a is disposed on the inside of the end opening 38, and helically engaged with the male thread 54a, to retain the end sleeve 54 at the end opening 38. A peripheral groove 54b is formed at a remaining tip of the end sleeve 54. An internal projection 58 or coupling claw is formed with an end of the closure device 56, disposed on a receiving groove in the closure device 56, shaped in an annular form or plural claw forms, and engaged with the peripheral groove 54b. When the closure device 56 is pressed to the end sleeve 54, the internal projection 58 is deformed resiliently and coupled to the peripheral groove 54b by snap-fit. Thus, the closure device 56 is tightly coupled to the end sleeve 54.

The closure device 56 includes a port housing 60, a port plate 62 for entry, and a sealing unit 64 or valve. The port housing 60 includes a housing sleeve 66 and a peripheral ring 68 (cover band) disposed around a tip of the housing sleeve 66. The internal projection 58 is formed on the housing sleeve 66, and surrounded by the peripheral ring 68.

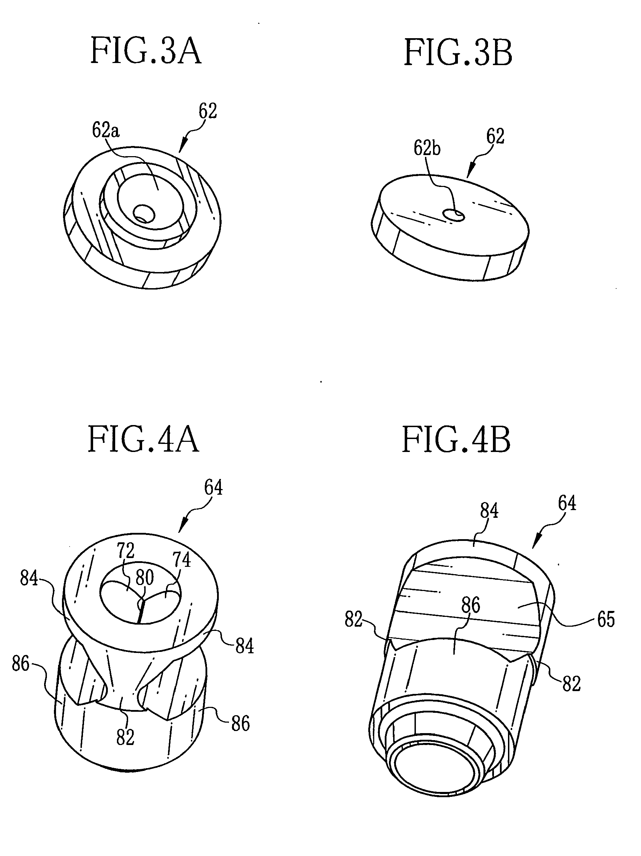

The port plate 62 and the sealing unit 64 are disposed in the housing sleeve 66. In Figs. 3A and 3B, the port plate 62 is formed from elastic material, such as silicone, and includes a tapered surface 62a and a circular access hole 62b. The tapered surface 62a is conically inclined. Also, the port plate 62 is disposed on a proximal side of the sealing unit 64. The tapered surface 62a is directed in the proximal direction. The medical instrument 40 is guided by the tapered surface 62a. A guide lumen 70 receives entry of the medical instrument 40 at its center.

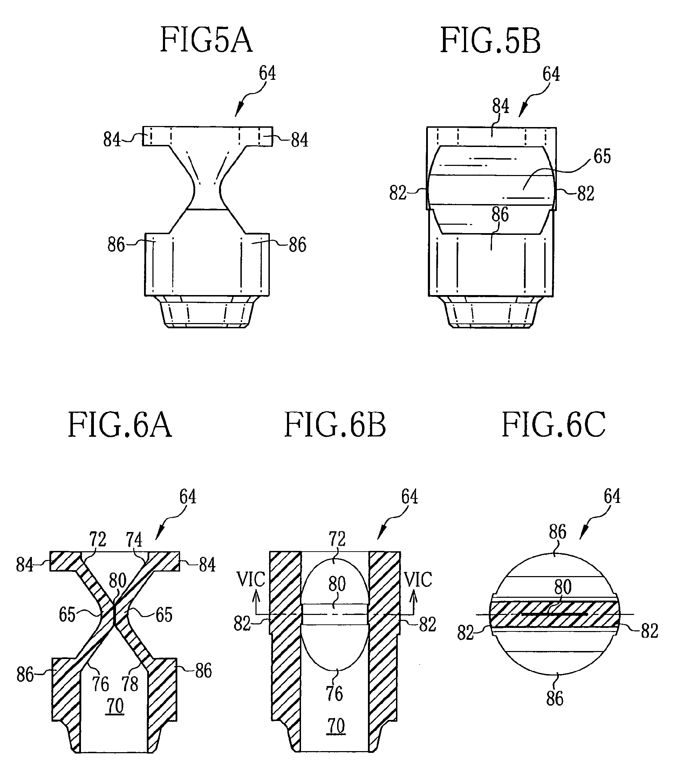

In Figs. 4A, 4B, 5A, 5B and 6A-6C, the sealing unit 64 is shaped in a sleeve form, and is formed from elastic material, such as silicone. Fig. 6C is a cross section taken on line VIC-VIC in Fig. 6B. The guide lumen 70 extends through the sealing unit 64. A pair of sealing walls 65 are provided in the sealing unit 64. Sealing surfaces of the sealing walls 65 contact one another to close the guide lumen 70 in their closed position.

Inclined surfaces 72 and 74 are formed with the sealing walls 65 of the sealing unit 64, and extend from the sealing surfaces in a proximal direction to increase a width of the guide lumen 70. Inclined surfaces 76 and 78 are formed with the sealing walls 65, and extend from the sealing surfaces in a distal direction to increase the width of the guide lumen 70. Thus, a slit opening 80 is defined by the sealing surfaces of an open position between the inclined surfaces 72 and 74 and the inclined surfaces 76 and 78.

When the medical instrument 40 is not present, the slit opening 80 is closed in the sealing unit 64. A tip of the medical instrument 40 accesses and presses open the slit opening 80 between the inclined surfaces 72 and 74. Two reinforcing walls 82 are formed by enlarging a thickness of lateral portions of the slit opening 80, for preventing breakage of the slit opening 80 in the course of entry of the medical instrument 40.

In the sealing unit 64, a coupling sleeve 86 or projection or support sleeve has a proximal end from which the sealing walls 65 project. A coupling flange 84 or projection is disposed annularly and extends from peripheral ends of the sealing walls 65 of the sealing unit 64. A first regulating projection 88 projects from an inner surface of the housing sleeve 66, and prevents the coupling flange 84 from moving in a distal direction. A second regulating projection 90 projects from an inner surface of the housing sleeve 66, and prevents the coupling sleeve 86 from moving in a proximal direction. See Fig. 2. The regulating projections 88 and 90 are disposed between the coupling flange 84 and the coupling sleeve 86 in Fig. 2, to construct the closure device 56 compactly without an excessive length.

A radial size of the first regulating projection 88 in the inward direction is larger than a radial size of the coupling flange 84 in the outward direction. The first regulating projection 88 presses the sealing unit 64 in a direction to close the slit opening 80. See Fig. 2. Similarly, a radial size of the second regulating projection 90 is larger than a radial size of the coupling sleeve 86. The second regulating projection 90 presses the sealing unit 64 in a direction to close the slit opening 80. See Fig. 2. This structure is effective in preventing leakage of fluid through the slit opening 80.

An internal space 92 is defined between the regulating projections 88 and 90 for allowing the sealing walls 65 of the sealing unit 64 to spread in outward directions upon entry of the medical instrument 40 in the guide lumen 70. See Fig. 2. In the present embodiment, an outer shape of the sealing unit 64 is an X-shape with a reduced width at the middle in correspondence with an inner surface of the sealing unit 64.

Accordingly, it is possible in the closure device 56 to prevent unwanted deformation of the sealing unit 64 because the first and second inclined surfaces operate to change the inner width, and proximal and distal ends of the sealing unit 64 are kept from moving by regulation. Also, the regulating projections are used for the regulation between the proximal and distal ends of the sealing unit 64, so that the sealing unit 64 can be longitudinally compact without an excessive length.

It is possible to enter the medical instrument 40 easily, because the internal space is maintained outside the sealing unit 64 in the closure device 56 to allow the sealing unit 64 to spread. The internal space is set large because the outer shape of the sealing unit 64 is an X-shape with a reduced width at the middle in correspondence with an inner surface of the sealing unit 64. Entry of the medical instrument 40 can be still easier. Also, the plug device can be compact in comparison with a comparative structure in which an internal space is formed by locally projecting a port housing for the purpose.

In the closure device 56, the sealing walls are pressed on one another to close the slit opening, so that leakage of fluid can be prevented reliably. It is possible to reduce a size of the closure device 56 in comparison with a structure in which a portion for regulating movement of the sealing walls is separate from a portion for tightly sealing a hole opening.

In the closure device 56, the sealing walls 65 are used to define the slit opening by spacing between their sealing surfaces. The sealing walls 65 receive the medical instrument 40. Thus, force to push open the slit opening is exerted in a side direction perpendicular to a diameter direction without exertion in the diameter direction, so that the entry of the medical instrument 40 is facilitated.

It is possible with the closure device 56 to prevent a problem of breakage of the slit opening 80 due to shortage of an expansion amount of the slit opening 80, owing to the shape of the slit opening 80. When the medical instrument 40 is not present, the slit opening 80 is closed. For passage of the medical instrument 40, the slit opening 80 is spread to such an extent that an inner diameter of the slit opening 80 becomes equal to an outer diameter of the medical instrument 40. If a tapered surface of a funnel shape is used to receive the medical instrument 40 in place of the above-described flat surfaces, a hole opening is circular. When the medical instrument 40 is not present, an inner diameter of the circular hole opening is as small as zero. For passage of the medical instrument 40, the inner diameter of the circular hole opening increases to become equal to the outer diameter of the medical instrument 40. The circular hole opening is likely to break due to increase of the expansion amount. However, in the invention, the slit opening 80 is considerably free from breakage, because the expansion amount of the slit opening 80 can be smaller than that of the circular hole opening.

The invention is not limited to the above embodiment. The port housing, although constituted by the housing sleeve and the peripheral ring in the above embodiment, can be a single piece including portions of the housing sleeve and the peripheral ring. In the above embodiment, the end sleeve is initially separate from the closure device or the end opening. However, the closure device or the end opening can be formed together with the end sleeve.

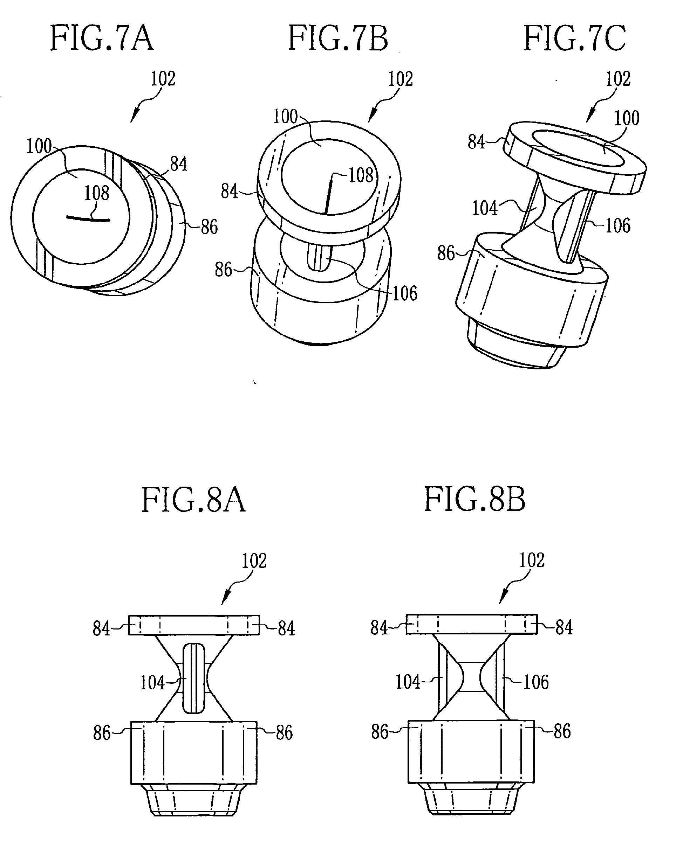

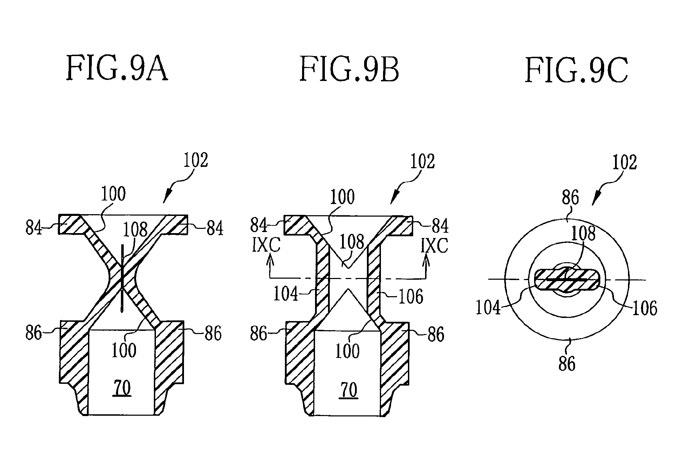

Although the sealing walls 65 are used in the above embodiment for sealing, other structures can be used in the invention. In Figs. 7A-7C, 8A, 8B and 9A-9C, one preferred sealing unit 102 or valve is illustrated, and includes a tapered surface 100 for receiving the medical instrument 40. Fig. 9C is a cross section taken on line IXC-IXC in Fig. 9B. Elements similar to those of the above embodiment are designated with identical reference numerals.

In Figs. 7A-9C, the sealing unit 102 has the tapered surface 100 oriented in directions opposite to one another. An outer surface of the sealing unit 102 is shaped in a manner corresponding to its inner surface. Ridge portions 104 and 106 or extensions are formed with the outer surface of the sealing unit 102. A slit opening 108 is defined through the medical instrument 40 by use of areas of the ridge portions 104 and 106. The ridge portions 104 and 106 are walls extending from the center axis. The ridge portion 106 is disposed opposite to the ridge portion 104 with respect to the center axis of the sealing unit 102 parallel with the axial direction of the medical instrument 40. With the sealing unit 102, it is possible to obtain effects similar to those of the above embodiment. In the present embodiment, the sealing unit has the shape in which two tapered surfaces of a conical shape are oriented in directions opposite to one another. However, a sealing unit can have a shape in which two tapered surfaces of a shape of a frustum of a cone, a hemispherical shape, and the like are oriented in directions opposite to one another.

In the closure device 56 of the above embodiments, a distal end of the coupling sleeve 86 is engaged with the inside of the end sleeve 54. The internal projection 58 or a distal end of the port housing 60 is engaged with the outside of the end sleeve 54, so that the closure device 56 is mounted on the end sleeve 54 in a plug-in fashion. However, the closure device 56 can have a plug form of which a distal end of a support sleeve is engaged only with the inside of the end sleeve 54, or can have a cap form of which a distal end of a support sleeve is engaged only with the outside of the end sleeve 54.

Methods of assembling parts of the embodiments are briefly described. To combine the housing sleeve 66 with the sealing unit 64, a center opening in the housing sleeve 66 can be widened resiliently, to fit the housing sleeve 66 on the outside of the sealing unit 64 in the axial direction. Also, the housing sleeve 66 can be constituted by two originally separate parts, which can be fitted on the outside of the sealing unit 64 and coupled to one another.

To combine the peripheral ring 68 (cover band) with the housing sleeve 66, a center opening in the peripheral ring 68 is resiliently widened to fit the peripheral ring 68 on the outside of the housing sleeve 66 in the axial direction. Also, the peripheral ring 68 can be constituted by two originally separate parts, which can be fitted on the outside of the housing sleeve 66 and coupled to one another.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A closure device for an end opening (38) of an instrument channel (50) for entry of an instrument (40), comprising:
a coupling sleeve (86) for connection with said end opening, said coupling sleeve having a guide lumen (70) for passage of said instrument;
at least two sealing walls (65), disposed to project from said coupling sleeve in a proximal direction in relation to said entry of said instrument, shiftable between open and closed positions, for tightly contacting one another to seal said guide lumen when in said closed position, said sealing walls being shifted in said open position by said instrument being entered, for preventing fluid from passing around said instrument;
a housing sleeve (60, 66) for containing at least said sealing walls;
a coupling flange (84), disposed at an end of said sealing walls in said proximal direction, for mounting said sealing walls in said housing sleeve;
a first regulating projection (90), disposed to project from an inner surface of said housing sleeve, for regulating movement of said coupling sleeve in said proximal direction;
a second regulating projection (88), disposed to project from said inner surface of said housing sleeve, for regulating movement of said coupling flange in a distal direction;
wherein each of said sealing walls includes:
a sealing surface for contact in relation to said closed position;
a first inclined surface disposed to extend from said sealing surface to said coupling sleeve;
a second inclined surface, disposed to extend from said sealing surface to said coupling flange, for guiding said instrument.

2. A closure device as defined in claim 1, wherein said second inclined surface is substantially flat, and an inner width thereof between said sealing walls increases toward said coupling flange.

3. A closure device as defined in claim 1, wherein said second inclined surface is curved, and an inner diameter thereof increases toward said coupling flange.

4. A closure device as defined in claim 1, wherein said sealing walls and said coupling sleeve constitute a sealing unit.

5. A closure device as defined in claim 1, further comprising an internal space, disposed between said housing sleeve and said sealing walls, for allowing said sealing walls to shift to said open position.

6. A closure device as defined in claim 5, wherein said first regulating projection is disposed on a distal side from said sealing surface, said second regulating projection is disposed on a proximal side from said sealing surface, and said internal space is defined between said first and second regulating projections.

7. A closure device as defined in claim 5, further comprising a recess, formed in said sealing walls with an outer surface back to said sealing surface, for constituting said internal space.

8. A closure device as defined in any one of claims 1-7, further comprising a pressing means for pressing said sealing walls toward said closed position.

9. A closure device as defined in claim 8, wherein said pressing means is said first regulating projection.

10. A closure device as defined in claim 8, wherein said pressing means is said second regulating projection.

11. A closure device as defined in any one of claims 1-7, further comprising a pair of reinforcing walls, disposed on lateral sides of said plural sealing walls, for preventing occurrence of breakage by spacing between said sealing walls according to entry of said instrument.

12. A closure device as defined in any one of claims 1-7, further comprising:
a port plate disposed in said housing sleeve on said proximal side of said sealing walls;
an access hole, formed in said port plate, for receiving passage of said instrument; and
an inclined surface, formed with said access hole to decrease an inner width toward said sealing walls, for guiding said instrument.

13. A closure device as defined in any one of claims 1-7, wherein said end opening has an end sleeve;
further comprising a receiving groove, disposed between said housing sleeve and said coupling sleeve, formed at a distal end thereof, for receiving a sleeve edge of said end sleeve.

14. A closure device as defined in claim 13, wherein said end opening further includes a peripheral groove formed around said end sleeve and near to a proximal end thereof;
further comprising an internal projection, formed on said housing sleeve to project from an inner surface of said receiving groove, engaged with said peripheral groove, for coupling to said end opening.

## Patentansprüche

1. Verschlussvorrichtung für eine Endöffnung (38) eines Instrumentenkanals (50) zum Eintritt eines Instruments (40), umfassend:
eine Kupplungshülse (86) zum Verbinden mit der Endöffnung, wobei die Kupplungshülse ein Führungslumen (70) für den Durchgang des Instruments aufweist;
mindestens zwei Dichtungswände, angeordnet, um von der Kupplungshülse in proximaler Richtung bezüglich des Eintritts des Instruments vorzustehen, verschiebbar zwischen einer Offenstellung und einer geschlossenen Stellung, um einander eng zu berühren und das Führungslumen in der geschlossenen Stellung abzudichten, wobei die Dichtungswände von dem eintretenden Instrument in die Offenstellung verschoben werden, um zu verhindern, dass Fluid in der Umgebung des Instruments hindurchtritt;
eine Gehäusehülse (60, 66) zur Aufnahme zumindest der Dichtungswände;
einen Kupplungsflansch (84), der an einem Ende der Dichtungswände in der proximalen Richtung angeordnet ist, um die Dichtungswände innerhalb der Gehäusehülse zu lagern;
einen ersten Reguliervorsprung (90), angeordnet, um von einer Innenfläche der Gehäusehülse vorzustehen, um eine Bewegung der Kupplungshülse in der proximalen Richtung zu regulieren;
einen zweiten Reguliervorsprung (88), angeordnet, um von der Innenfläche der Gehäusehülse abzustehen und eine Bewegung des Kupplungsflansches in einer distalen Richtung zu regulieren,
wobei jede der Dichtungswände enthält:
eine Dichtungsfläche zur Berührung in Verbindung mit der geschlossenen Stellung;
eine erste geneigte Fläche, angeordnet zur Erstreckung von der Dichtungsfläche zu der Kupplungshülse;
eine zweite geneigte Fläche, angeordnet zur Erstreckung von der Dichtungsfläche zu dem Kupplungsflansch, um das Instrument zu führen.

2. Verschlussvorrichtung nach Anspruch 1, bei der die zweite geneigte Fläche im wesentlichen flach ist, und ihre innere Breite zwischen den Dichtungswänden in Richtung des Kupplungsflansches zunimmt.

3. Verschlussvorrichtung nach Anspruch 1, bei der die zweite geneigte Fläche gekrümmt ist, und ihr Innendurchmesser in Richtung zu dem Kupplungsflansch hin zunimmt.

4. Verschlussvorrichtung nach Anspruch 1, bei der die Dichtungswände und die Kupplungshülse eine Dichtungseinheit bilden.

5. Verschlussvorrichtung nach Anspruch 1, weiterhin umfassend einen Innenraum, angeordnet zwischen der Gehäusehülse und den Dichtungswänden, damit die Dichtungswände in die Offenstellung verschoben werden können.

6. Verschlussvorrichtung nach Anspruch 5, bei der der erste Reguliervorsprung sich an einer distalen Seite bezüglich der Dichtungsfläche befindet, der zweite Reguliervorsprung sich an einer proximalen Seite bezüglich der Dichtungsfläche befindet, und der Innenraum zwischen dem ersten und dem zweiten Reguliervorsprung ausgebildet ist.

7. Verschlussvorrichtung nach Anspruch 5, weiterhin umfassend eine Ausnehmung, die zur Bildung des Innenraums in den Dichtungswänden mit einer zu der Dichtungsfläche zurückgesetzten Außenfläche ausgebildet ist.

8. Verschlussvorrichtung nach einem der Ansprüche 1 bis 7, weiterhin umfassend eine Presseinrichtung zum Pressen der Dichtungswände in die geschlossene Stellung.

9. Verschlussvorrichtung nach Anspruch 8, bei der die Presseinrichtung durch den ersten Reguliervorsprung gebildet ist.

10. Verschlussvorrichtung nach Anspruch 8, bei der die Presseinrichtung durch den zweien Reguliervorsprung gebildet ist.

11. Verschlussvorrichtung nach einem der Ansprüche 1 bis 7, weiterhin umfassend ein Paar Verstärkungswände, angeordnet auf Seitenbereichen der mehreren Dichtungswände, um das Auftreten eines Bruchs aufgrund einer Beabstandung zwischen den Dichtungswänden bei Eintritt des Instruments zu verhindern.

12. Verschlussvorrichtung nach einem der Ansprüche 1 bis 7, weiterhin umfassend:
eine in der Gehäusehülse auf der proximalen Seite der Dichtungswände angeordnete Öffnungsplatte;
ein in der Öffnungsplatte ausgebildetes Zugangsloch zum Aufnehmen des Instrumentendurchgangs; und
eine geneigte Fläche, die bei dem Zugangsloch ausgebildet ist, um eine innere Breite in Richtung der Dichtungswände zu verringern, um das Instrument zu führen.

13. Verschlussvorrichtung nach einem der Ansprüche 1 bis 7, bei der die Endöffnung eine Endhülse aufweist;
weiterhin umfassend eine Aufnahmenut, angeordnet zwischen der Gehäusehülse und der Kupplungshülse, ausgebildet an ihrem distalen Ende, um einen Hülsenrand der Endhülse aufzunehmen.

14. Verschlussvorrichtung nach Anspruch 13, bei der die Endöffnung weiterhin eine Umfangsnut aufweist, die um die Endhülse herum und in der Nähe ihres proximalen Endes ausgebildet ist;
weiterhin umfassend einen Innenvorsprung, der an der Gehäusehülse ausgebildet ist, um von einer Innenfläche der Aufnahmenut abzustehen, zusammenwirkend mit der Umfangsnut, um die Endöffnung zu koppeln.

## Revendications

1. Dispositif de fermeture destiné à une ouverture d'extrémité (38) d'un canal d'instrument (50) pour l'entrée d'un instrument (40), comprenant :
un manchon d'accouplement (86) destiné à un raccordement avec ladite ouverture d'extrémité, ledit manchon d'accouplement présentant une lumière de guidage (70) pour le passage dudit instrument ;
au moins deux parois d'étanchéité (65), disposées de manière à faire saillie depuis ledit manchon d'accouplement dans une direction proximale par rapport à ladite entrée dudit instrument, pouvant être déplacées entre des positions ouverte et fermée, afin d'être étroitement en contact l'une avec l'autre pour sceller ladite lumière de guidage lorsque qu'elles se trouvent dans ladite position fermée, lesdites parois d'étanchéité étant déplacées dans ladite position ouverte par ledit instrument en cours d'introduction, afin d'empêcher le passage de fluide autour dudit instrument ;
un manchon de logement (60, 66) destiné à contenir au moins lesdites parois d'étanchéité ;
une bride d'accouplement (84), disposée au niveau d'une extrémité desdites parois d'étanchéité dans ladite direction proximale, destinée au montage desdites parois d'étanchéité dans ledit manchon de logement ;
une première saillie régulatrice (90), disposée de manière à faire saillie à partir d'une surface intérieure dudit manchon de logement, destinée à réguler le déplacement dudit manchon d'accouplement dans ladite direction proximale ;
une seconde saillie régulatrice (88), disposée de manière à faire saillie à partir de ladite surface intérieure dudit manchon de logement, destinée à réguler le déplacement de ladite bride d'accouplement dans une direction distale ;
dans lequel chacune desdites parois d'étanchéité inclut :
une surface d'étanchéité pour un contact lié à ladite position fermée ;
une première surface inclinée disposée de manière à s'étendre de ladite surface d'étanchéité audit manchon d'accouplement ;
une seconde surface inclinée, disposée de manière à s'étendre de ladite surface d'étanchéité à ladite bride d'accouplement, destinée à guider ledit instrument.

2. Dispositif de fermeture selon la revendication 1, dans lequel ladite seconde surface inclinée est sensiblement plate, et une largeur intérieure de celle-ci entre lesdites parois d'étanchéité augmente en direction de ladite bride d'accouplement.

3. Dispositif de fermeture selon la revendication 1, dans lequel ladite seconde surface inclinée est incurvée, et un diamètre intérieur de celle-ci augmente en direction de ladite bride d'accouplement.

4. Dispositif de fermeture selon la revendication 1, dans lequel lesdites parois d'étanchéité et ledit manchon d'accouplement constituent une unité d'étanchéité.

5. Dispositif de fermeture selon la revendication 1, comprenant en outre un espace interne, disposé entre ledit manchon de logement et lesdites parois d'étanchéité, afin de permettre auxdites parois d'étanchéité de se déplacer vers ladite position ouverte.

6. Dispositif de fermeture selon la revendication 5, dans lequel ladite première saillie régulatrice est disposée sur un côté distal à partir de ladite surface d'étanchéité, ladite seconde saillie régulatrice est disposée sur un côté proximal à partir de ladite surface d'étanchéité, et ledit espace interne est défini entre lesdites première et seconde saillies régulatrices.

7. Dispositif de fermeture selon la revendication 5, comprenant en outre un retrait, formé dans lesdites parois d'étanchéité avec une surface extérieure retournant vers ladite surface d'étanchéité, afin de constituer ledit espace interne.

8. Dispositif de fermeture selon l'une quelconque des revendications 1 à 7, comprenant en outre un moyen de pression destiné à presser lesdites parois d'étanchéité vers ladite position fermée.

9. Dispositif de fermeture selon la revendication 8, dans lequel ledit moyen de pression est ladite première saillie régulatrice.

10. Dispositif de fermeture selon la revendication 8, dans lequel ledit moyen de pression est ladite seconde saillie régulatrice.

11. Dispositif de fermeture selon l'une quelconque des revendications 1 à 7, comprenant en outre une paire de parois de renfort, disposées sur des côtés latéraux desdites plusieurs parois d'étanchéité, afin d'empêcher l'apparition d'une rupture par l'espacement entre lesdites parois d'étanchéité lors de l'introduction dudit instrument.

12. Dispositif de fermeture selon l'une quelconque des revendications 1 à 7, comprenant en outre :
une plaque d'entrée disposée dans ledit manchon de logement sur ledit côté proximal desdites parois d' étanchéité ;
un trou d'accès, formé dans ladite plaque d'entrée, destiné à recevoir le passage dudit instrument, et
une surface inclinée, formée avec ledit trou d'accès afin de diminuer une largeur intérieure vers lesdites parois d'étanchéité, afin de guider ledit instrument.

13. Dispositif de fermeture selon l'une quelconque des revendications 1 à 7, dans lequel ladite ouverture d'extrémité comporte un manchon d'extrémité ;
comprenant en outre une rainure de réception, disposée entre ledit manchon de logement et ledit manchon d'accouplement, formée au niveau d'une extrémité distale de ceux-ci, afin de recevoir un bord de manchon dudit manchon d'extrémité.

14. Dispositif de fermeture selon la revendication 13, dans lequel ladite ouverture d'extrémité inclut en outre une rainure périphérique formée autour dudit manchon d'extrémité et à proximité d'une extrémité proximale de celui-ci ;
comprenant en outre une saillie interne, formée sur ledit manchon de logement afin de faire saillie à partir d'une surface interne de ladite rainure de réception, en prise avec ladite rainure périphérique, pour un accouplement avec ladite ouverture d'extrémité.
